Europäisches Patentamt

European Patent Office

Office européen des brevets

(19)

(11) Numéro de publication : **0 243 322 B1**

(12) **FASCICULE DE BREVET EUROPEEN**

(45) Date de publication du fascicule du brevet :
**29.01.92 Bulletin 92/05**

(51) Int. Cl.⁵ : **C07K 15/00, A61K 37/14,**
**// C07K3/08**

(21) Numéro de dépôt : **87830097.9**

(22) Date de dépôt : **16.03.87**

(54) **Composés contenant du fer biodisponible, procédé pour leur préparation et compositions pharmaceutiques qui les contiennent.**

Le dossier contient des informations techniques présentées postérieurement au dépôt de la demande et ne figurant pas dans le présent fascicule.

(30) Priorité : **18.03.86 IT 1978786**

(43) Date de publication de la demande :
**28.10.87 Bulletin 87/44**

(45) Mention de la délivrance du brevet :
**29.01.92 Bulletin 92/05**

(84) Etats contractants désignés :
**BE DE ES FR GB NL SE**

(56) Documents cités :
**GB-A- 2 115 821
BIOLOGICAL ABSTRACTS, vol. 75, no. 9, 1983, résumé no. 62206, Philadelphia, PA, US; M.J. McKAY et al.: "ATP-independent proteolysis of globin cyanogen bromide peptides in rabbit reticulocyte cell-free extracts", & EUR. J. BIOCHEM. 125(3): 567-574. 1982**

(56) Documents cités :
**CHEMICAL ABSTRACTS, vol. 88, no. 3, 16 janvier 1978, page 485, résumé no. 21017t, Columbus, Ohio, US; M. FRIEDMAN et al.: "Protected proteins in ruminant nutrition. In vitro evaluation of casein derivatives", & ADV. EXP. MED. BIOL. 1977, 86B, 545-58
CHEMICAL ABSTRACTS, vol. 90, no. 21, 21 mai 1979, page 468, résumé no. 166859v, Columbus, Ohio, US; E.R. MILLER et al.: "Evaluation of iron proteinate for indirect prevention of baby pig anemia", & TRACE ELEM. METAB. MAN ANIM., PROC. INT. SYMP., 3rd 1977 (Pub. 1978), 506-10**

(73) Titulaire : **MAGIS FARMACEUTICI S.P.A.
Via Cacciamali 34/36/38
I-25100 Brescia (IT)**

(72) Inventeur : **Puricelli, Laura
Via Taramelli, 7
I-25128 Brescia (IT)**

(74) Mandataire : **Gervasi, Gemma et al
NOTARBARTOLO & GERVASI Srl Viale Bianca Maria 33
I-20122 Milan (IT)**

# Description

Le fer est un constituant essentiel du sang, du fait qu'il est nécessaire à la formation de l'hémoglobine et aux processus d'oxydation pour la vie des tissus. Le sang contient environ 3,5 g de fer, dont les deux tiers sont présents sous forme d'hémoglobile et le restant tiers en tant que fer emmagasiné dans le système réticulo-endothélial.

Le fer est surtout absorbé par le duodénum et par la partie supérieure du jéjunum où l'absorption a lieu à l'aide de la sécrétion acide de l'estomac. Seulement une petite portion, probablement moins d'un dixième du fer ingéré avec les aliments, est absorbée mais il paraît que cette quantité soit suffisante à un équilibre normal du fer dans les adultes.

Les anémies par carences de fer sont souvent le résultat de graves hémorragies chroniques, pauvreté alimentaire, grossesse et infections provoquées par les parasites. Les anémies dues à carences en fer répondent facilement à la thérapeutique du fer.

Des dérivés du fer sont utilisés pour le traitement de toutes les formes d'anémie microcytique, y compris la simple anémie achlorhydrique, la simple anémie des enfants, l'anémie due à une hémorragie excessive et l'anémie associée aux infections, notamment les infections provoquées par les parasites et les maladies malignes.

L'administration par voie orale de préparations à base de fer cause souvent des irritations gastro-intestinales suivies de vomissement et de diarrhée. Des administrations continuelles produisent souvent des constipations.

Pour cette raison la thérapeutique du fer avec les différents produits répandus dans le commerce, tels que les sels organiques du fer, comme par exemple le citrate, le cholinate, l'aspartate, le fumarate, le gluconate, le glycinate, le lactate et ainsi de suite, ainsi qu'avec les sels inorganiques comme par exemple le sulfate, le bisulfate, le chlorure, ne donne pas de résultats satisfaisants du fait que l'absorption peut être influencée par l'irritation gastro-intestinale causée par ces mêmes produits.

La demande de brevet GB-A-2 115 821 décrit protéines succinylées, avec un degré de succinylation compris entre 20 et 100%, contenant entre 0.1 et 20% en poids de fer, obtenues en traitant des protéines succinylées avec des sels de fer en solution aqueuse à pH entre 2 et 10. Ces composés sont bien tolérés et ils ont une haute biodisponibilité du fer.

Le but de la présente invention était de mettre à la disposition de nouvelles compositions contenant du fer biodisponible qui étaient dépourvues des désavantages mentionnés plus haut et qui pouvaient être préparées d'une manière simple et susceptible d'être reproduite.

Ce but a été atteint de manière surprenante par la découverte que les protéines d'origine végétale et animale peuvent acquérir des groupes carboxyliques en mesure de se combiner au fer fournissant de cette façon des dérivés ayant un contenu élevé en fer, facilement absorbables et tolérables et dépourvus d'effets collatéraux dûs a d'éventuelles lésions gastriques.

La présente invention a donc pour objet des protéines modifiées moyennant l'introduction de groupes carboxyliques, les dérivés avec le fer des protéines ainsi modifiées, un procédé de préparation des protéines modifiées et leurs dérivés avec le fer, ainsi que des compositions pharmaceutiques appropriées au traitement de maladies dues à des carences de fer et qui contiennent ces dérivés du fer en tant que principe actif.

Les groupes acides peuvent être introduits dans les protéines en les faisant entrer en réaction de manière appropriée avec un anhydride d'un acide bicarboxylique aliphatique choisi entre acide acétylaspartique et acide acétylglutamique.

Les protéines en état d'être transformées par l'introduction de groupes carboxyliques selon la présente invention sont généralement des protéines d'origine végétale et animale, par exemple les protéines du lait en poudre, du lait précipité, de l'oeuf, du sérum bovin, de soja, de foie, d'extraits de viande de volatiles, de poissons et d'animaux. On fait normalement réagir les protéines mentionnées ci-dessus sous forme de leurs lysats partiels.

En vue d'obtenir ces protéines modifiées, elles sont solubilisées dans l'eau et la solution ainsi obtenue est portée à un pH de 7,5 à 8,0 moyennant l'adjonction d'une base, par exemple de l'hydrate sodique, du carbonate ou bicarbonate sodique ou un autre sel alcalin.

En agitant et en contrôlant soigneusement le pH, on ajoute ensuite à la solution alcaline, en petites portions, l'anhydride de l'acide bicarboxylique désiré. Les groupes acides qui se combinent varient en pourcentage suivant la quantité d'anhydride qui est ajouté à la protéine.

On fait ensuite réagir les nouveaux dérivés des protéines ainsi obtenues avec un sel soluble de fer, par exemple du chlorure ferrique, à donner des sels de fer biodisponible qui, administrés par voie orale, ne produisent pas d'irritations gastro-intestinales. Le groupe acide introduit dépendra évidemment, comme précisé plus haut, de la quantité d'anhydride qui se combine aux groupes aminés libres de la protéine, de sorte qu'il y

2

aura également une variation en ce qui concerne la teneur en fer qui se combine. Généralement la teneur en fer des nouveaux composés variera depuis 0,1% jusqu'à 20% en poids.

A partir de ces composés on peut ensuite préparer de manière connue des compositions pharmaceutiques appropriées à la thérapeutique d'anémies de sidéropénie, lesquelles se présentent de préférence sous forme de sachets à une dose, sirops, ampoules ou petits flacons buvables, comprimés, capsules ou dragées.Chaque composition pharmaceutique contiendra, outre le principe actif, également les adjuvants et/ou additifs habituels appropriés à la préparation des compositions énumérées ci-dessus.

Les exemples qui suivent servent à illustrer le procédé de préparation des nouveaux composés faisant l'objet de l'invention, mais ils ne limitent aucunement l'essence la plus intime de l'invention.

EXEMPLE 1 - Préparation de dérivés de fer à partir de protéines de soja maléilées.

10 g de protéines de soja sont suspendus en 180 ml d'eau et on ajoute ensuite, en agitant, du bicarbonate de sodium en poudre jusqu'à solution complète. On contrôle le pH qui doit varier entre 7,5 et 8,0. En petites portions, sous contrôle pH-métrique compris entre 7,5 et 8, on ajoute ensuite 5,1 g d'anhydride maléique. On agite encore pendant trois heures, puis on corrige le pH jusqu'à une valeur de $3 \pm 0,5$. Le précipité qui se forme est filtré et lavé avec de l'eau. Le précipité humide est ensuite dissous en 150 ml d'eau et porté jusqu'à un pH de 7,5 - 8 avec du carbonate de sodium. On filtre la solution obtenue et on acidifie avec de l'acide chlorhydrique jusqu'à pH 3,0.

Le précipité obtenu est centrifugé et séché en étuve. On pèse le précipité obtenu et on le suspend dans l'eau de manière à avoir un concentration de protéines d'environ 0,03 g/litre; on porte ensuite sous forme de solution avec de l'hydrate de sodium jusqu'à pH 7,5 - 8,0. A ce point on ajoute du chlorure ferrique en solution, en quantité suffisante à permettre que le rapport entre $Fe^{3+}$ et protéine maléilée soit de $1 \div 10$ en poids. Le pH descend à $2,5 \pm 0,2$. On obtient une suspension qui est agitée pendant trois heures à température ambiante et ensuite centrifugée. Le produit solide récupéré est suspendu dans l'eau, on corrige le pH à 7,5 avec de l'hydrate de sodium, on filtre et on corrige encore le pH à 2,5 avec de l'acide chlorhydrique.

Le précipité qui se forme est centrifuge, lavé avec de l'eau désionisée jusqu'à l'élimination du sel et ensuite séché en étuve.

Le rendement est d'environ 18% de la protéine utilisée. En alternative le produit peut être isolé de la solution aqueuse à pH 7,5, après filtrage, qui est dialysée dans le but d'éliminer le chlorure de sodium; on porte ensuite à sec par lyophilisation et Spray Dryer.

EXEMPLE 2

Par la même procédure de l'exemple 1 on peut obtenir des dérivés du fer de protéines maléilées obtenues des protéines de soja partiellement hydrolysées,des protéines du lait en poudre intégrales ou partiellement hydrolysées, des protéines précipitées du lait intégrales ou partiellement hydrolysées, des protéines de l'oeuf intégrales ou partiellement hydrolysées, des protéines du serum bovin intégrales ou partiellement hydrolysées, des protéines du foie intégrales ou partiellement hydrolysées, des protéines de la viande provenant d'animaux domestiques, oiseaux ou poissons, intégrales ou partiellement hydrolysées.

Sur le tableau I on a reporté les caractéristiques des produits obtenus.

EXEMPLE 3

En employant la même procédure de l'exemple 1 et les protéines de différente provenance mentionnées dans l'exemple 2, mais en remplaçant l'anhydride maléique par l'anhydride acétylglutamique ou l'anhydride acétylaspartique, on obtient les composés reportés sur le tableau I, sur lequel on a également énuméré leurs caractéristiques principales.

EXEMPLE 4

Si l'on procède de la même manière que dans les exemples 1 à 3, en employant toutefois des quantités plus élevées de protéine par rapport à l'anhydride, on obtient des protéines ayant des quantités plus petites d'acide lié au groupe aminé, ce qui se traduit par des dérivés ayant un contenu variable en fer allant de 0,1% à 20% en poids.

3

TABLEAU I

| Produit | Matière première utilisée | Réactif | Rende- ment (%) | Conte- nu en fer (%) |
|---------|---------------------------|---------|-----------------|----------------------|

| | | | | |
|---|---|---|---|---|
| – Dérivés du fer de protéines de soja ou soja partiellement hydrolysé acétylglutamilées | Protéines de soja ou soja partiellement hydrolysé | Anhydride acétylglutamique | 20<br>21 | 4,8<br>5,2 |
| – Dérivés du fer de protéines du lait ou lait partiellement hydrolysé acétyl glutamilées | Lait en poudre ou partiellement hydrolysé | " | 20<br>21 | 6,1<br>6,8 |
| – Dérivés du fer de protéines du lait précipité ou partiellement hydrolysé acétylglutamilées | Lait précipité ou partiellement hydrolysé | " | 19<br>18 | 6<br>6,5 |
| – Dérivés du fer de protéines de sérum bovin ou sérum bovin partiellement hydrolysé acétyl glutamilées | Sérum bovin ou sérum bovin partiellement hydrolysé | " | 20<br>18 | 6,2<br>6,7 |
| – Dérivés du fer de protéines de foie ou foie partiellement hydrolysé acétylglutamilées | Protéines de foie ou foie partiellement hydrolysé | " | 21<br>20 | 8,5<br>9,7 |
| – Dérivés du fer de protéines de poisson ou poisson partiellement hydrolysé acétylglutamilées | Protéines de poisson ou poisson partiellement hydrolysé | " | 20<br>21 | 6,1<br>6,5 |

5

| | | | | |
|---|---|---|---|---|
| - Dérivés du fer de protéines de soja ou soja partiellement hydrolysé acétylaspartilées | Protéines de soja ou soja partiellement hydrolysé | Anhydryde acétylas partique | 22 | 4,9 |
| | | | 21 | 5,5 |
| - Dérivés du fer de protéines du lait en poudre ou partiellement hydrolysé acétylaspartilées | Lait en poudre ou partiellement hydrolysé | " | 20 | 5,1 |
| | | | 20 | 6,1 |
| - Dérivés du fer de protéines du lait précipité ou partiellement hydrolysé acétylaspartilées | Lait précipité ou partiellement hydrolysé | " | 20 | 4,8 |
| | | | 20 | 5,6 |
| - Dérivés du fer de protéines de sérum bovin ou sérum bovin partiellement hydrolysé acétyl aspartilées | Sérum bovin ou sérum bovin partiellement hydrolysé | " | 20 | 5,8 |
| | | | 21 | 6,3 |
| - Dérivés du fer de protéines de foie ou foie partiellement hydrolysé acétylaspartilées | Protéines de foie ou foie partiellement hydrolysé | " | 20 | 8,5 |
| | | | 21 | 9,5 |
| - Dérivés du fer de protéines de poisson ou poisson partiellement hydroly- | Protéines de poisson ou poisson partiellement | " | 20 | 4,9 |
| | | | 21 | 5,9 |

Caractéristiques chimico-physiques des dérivés des protéines du fer obtenus selon les exemples 1 à 4

Les caractéristiques chimico-physiques sont fournies:

a) d'après l'analyse des groupes acides qui se sont unis aux groupes aminés libres présents dans la protéine. Les groupes acides sont déterminés par la différence entre le titre du groupe aminé libre avant et après la formation du dérivé acide. La méthode appliquée est celle de la ninhydrine;

b) d'après le contenu en fer présent qui est déterminé par la méthode de l'O-phénanthroline;

c) d'après le tracé électrophorétique sur l'actétate de cellulose des dérivés en comparaison avec les protéines desquelles ils dérivent;

d) d'après la stabilité en suc gastrique qui est effectuée par la procédure qui suit : 30 mg du dérivé sont dissous en 0,5 ml d'eau, et on y ajoute ensuite 5 ml de suc gastrique. On obtient un précipité. La suspension est incubée à 37°C pendant 60 minutes, en agitant. On centrifuge et on titre le fer sur le filtrat. Le fer cédé au suc gastrique ne dépasse pas 1% du total initial. L'absence de fer non lié est démontré par la solubilité parfaite et complète des nouveaux composés en ambiance alcaline. En effet le fer non lié précipiterait sous forme d'hydroxyde de fer.

Caractéristiques pharmaco-biologiques des nouveaux dérivés

Toxicité aiguë

La DL50 déterminée dans la souris Swiss par administration par voie orale était supérieure à 4000 mg/kg.

Variations pondérales et consommation de nourriture

Des rats mâles Wistar ont été divisés en trois groupes, mis en stabulation pendant 14 jours avant le traitement, et ensuite séparés au hasard en groupes de 10.

Le premier groupe est utilisé en tant que contrôle. Le deuxième groupe est traité avec 100 mg/kg/jour de Ferritina (composé de fer de comparaison). Le troisième groupe est traité avec 100 mg/kg/jour de nouveaux dérivés.

Pendant le traitement les rats sont pesés chaque jour avant et après l'administration. La consommation de nourriture est mesurée à la fin du traitement. On a remarqué que l'augmentation pondérale a été ralentie de manière considérable dans les rats traités avec les nouveaux dérivés par rapport à ceux traités avec Ferratina et à ceux de contrôle. On a également remarqué une importante diminution de la consommation de nourriture dans les rats traités avec les nouveaux dérivés par rapport à ceux traités avec Ferratina et à ceux de contrôle.

Niveaux hématiques après administration par voie orale

On a effectué l'épreuve sur des rats mâles Wistar pesant environ 200 g, à jeun depuis 18 heures, divisés au hasard en groupes de 10. Chaque groupe a été sacrifié à l'heure établie et on a prélevé le sang à chaque animal. La détermination du fer a été effectuée par spectrophotométrie moyennant de l'acide $\beta$-phénanthrolinesulfonique.

Les épreuves ont été effectuées en comparaison avec du sulfate ferreux et Ferratina. La quantité de fer/kg était pour tous les dérivés d'un mg/kg. D'après les résultats obtenus on peut constater que les niveaux hématiques des nouveaux dérivés, par rapport à ceux du sulfate ferreux, se prolongent davantage avec le temps et par rapport à ceux de la Ferratina sont bien plus élevés d'environ (1,5 - 1).

**Revendications**

**Revendications pour les Etats contractants suivants : BE,DE,FR,GB,NL,SE**

1. Protéines d'origine végétale ou animale modifiées par l'introduction dans leur structure de groupes carboxyliques en mesure de se combiner au fer, caractérisées en ce que l'introduction des groupes carboxyliques a lieu par réaction des protéines avec un anhydride d'un acide bicarboxylique aliphatique choisi entre l'acide acétylaspartique et l'acide acétylglutamique.

2. Protéines selon la revendication 1, caractérisées en ce qu'elles sont choisies parmi les protéines du lait en poudre, du lait précipité ou partiellement hydrolysé, de l'oeuf, du sérum bovin, du foie, du poison, de soja, d'extraits de viande de volatiles et d'animaux.

3. Composés contenant du fer biodisponible obtenus par réaction des protéines modifiées des revendications 1 et 2 avec un dérivé du fer.

4. Composés selon la revendication 3, caractérisés en ce que le dérivé du fer est du chlorure ferrique.

5. Composés selon la revendication 3, caractérisés en ce que la teneur en fer dans ceux-ci est comprise entre 0,1 et 20% en poids.

6. Protéines selon la revendication 2, caractérisées en ce qu'elles peuvent être partiellement hydrolysées.

7. Procédé pour la préparation des protéines des revendications 1 et 2, caractérisé en ce qu'en contrôlant strictement la valeur du pH on fait entrer en réaction les protéines dissoutes dans l'eau avec un anhydride d'un acide bicarboxylique aliphatique choisi entre l'acide acétylaspartique et l'acide acétylglutamique.

8. Procédé selon la revendication 7, caractérisé en ce que le pH est maintenu à une valeur de 7,5-8,5 moyennant l'adjonction d'une base, où la base est un hydroxide alcalin ou un carbonate ou bicarbonate alcalin.

9. Procédé selon les revendications 7 et 8, caractérisé en ce que la base est un hydroxyde sodique, carbonate sodique ou bicarbonate sodique.

10. Procédé pour la préparation des protéines des revendications 3 à 5, charactérisé en ce que les protéines modifiées obtenues selon le procédé de la revendication 7, après leur isolement ou sans les isoler, sont traitées avec un dérivé du fer et le produit qui se forme est séparé par lyophilisation ou par Spray Dryer.

11. Procédé selon la revendication 10, caractérisé en ce que le dérivé du fer est chlorure ferrique.

12. Composition pharmaceutiques pour le traitement des anémies dues à carences en fer, caractérisées en ce qu'elles contiennent, en tant que principe actif, un composé selon les revendications 3 à 5.

13. Compositions pharmaceutiques selon la revendication 12 sous forme de sachets à une seule dose, sirops, ampoules ou petits flacons buvables, comprimés, capsules, dragées et similaires.

**Revendications pour l'Etat contractant suivant : ES**

1. Procédé pour la préparation des protéines d'origine végétale ou animale modifées par l'introduction dans leur structure de groupes carboxyliques en mesure de se combiner au fer, caractérisé en ce que l'introduction des groupes carboxyliques a lieu par réaction des protéines avec un anhydride d'un acide bicarboxylique aliphatique choisi entre l'acide acétylaspartique e l'acide acétylglutamique.

2. Procédé selon la revendication 1, caractérisé en ce que les protéines sont choisies parmi les protéines du lait en poudre, du lait précipité ou partiellement hydrolysé, de l'oeuf, du sérum bovin, du foie, du poisson, de soja, d'extraits de viande de volatiles et d'animaux.

3. Procédé selon les revendications 1 et 2, caractérisé en ce que les protéines peuvent être partiellement hydrolysées.

4. Procédé selon les revendications 1 à 3, caractérisé en ce qu'en contrôlant strictement la valeur du pH on fait entrer en réaction les protéines dissoutes dans l'eau avec un anhydride d'un acide bicarboxylique aliphatique choisi entre l'acide acétylaspartique et l'acide acètylglutamique.

5. Procédé selon la revendication 4, caractérisé en ce que le pH est maintenu à une valeur de 7,5-8,5 moyennant l'adjonction d'une base, où la base est un hydroxyde alcalin ou un carbonate ou bicarbonate alcalin.

6. Procédé selon les revendications 4 et 5, caractérisé en ce que la base est un hydroxyde sodique, carbonate sodique ou bicarbonate sodique.

7. Procédé pour la préparation des composés contenant du fer biodisponible, caractérisé en ce que les protéines modifiées obtenues selon le procédé des revendications 1 à 6, après leur isolement ou sans les isoler, sont traitées avec un dérivé du fer.

8. Procédé selon la revendication 7, caractérisé en ce que le dérivé du fer est chlorure ferrique.

9. Procédé selon les revendications 7 et 8, caractérisé en ce que le teneur en fer dans les composés contenant du fer biodisponible est comprise entre 0,1 et 20% en poids.

10. Procédé selon les revendications 7 à 9, caractérisé en ce que le produit qui se forme après le traitement des protéines modifiées avec un dérivé du fer est séparé par lyophilisation ou par Spray Dryer.

11. Procédé pour la préparation des compositions pharmaceutiques pour le traitement des anémies dues à carences en fer, caractérisé en ce que les composés contenant du fer obtenus selon le procédé des revendications 7 à 10 sont mêlés avec adjuvants et/ou additifs habituels.

**Claims**

**Claims for the following Contracting States : BE,DE,FR,GB,NL,SE**

1. Proteins of vegetable or animal origin modified by the introduction into their structure of carboxylic groups able to combine with iron, characterised in that the introduction of carboxylic groups takes place through reaction of the proteins with an anhydride of an aliphatic dicarboxylic acid chosen from acetylaspartic acid and acetylglutamic acid.

2. Proteins according to claim 1, characterised in that they are chosen from the proteins of powdered milk, precipitated or partially hydrolysed milk, egg, bovine serum, liver, fish, soya, extracts of bird and animal meat.

3. Compounds containing bioavailable iron which are obtained through reaction of the modified proteins of claims 1 and 2 with an iron derivative.

4. Compounds according to claim 3, characterised in that the iron derivative is ferric chloride.

5. Compounds according to claim 3, characterised in that the iron content in the said compounds is comprised between 0.1% and 20% by weight.

6. Proteins according to claim 2, characterised in that they can be partially hydrolysed.

7. A method of preparing the proteins of claims 1 and 2, characterised in that by strictly controlling the pH value, the proteins dissolved in water are reacted with an anhydride of an aliphatic dicarboxylic acid chosen from acetylaspartic acid and acetylglutamic acid.

8. A method according to claim 7, characterised in that the pH is maintained at a value of 7.5-8.5 by means of the addition of a base, where the base is an alkaline hydroxide or an alkaline carbonate or bicarbonate.

9. A method according to claims 7 and 8, characterised in that the base is a sodium hydroxide, sodium carbonate or sodium bicarbonate.

10. A method of preparing the proteins of claims 3 to 5, characterised in that the modified proteins obtained according to the method of claim 7, after their isolation or without isolating them, are treated with an iron derivative and the product which is formed is separated by lyophilisation or by spray dryer.

11. A method according to claim 10, characterised in that the iron derivative is ferric chloride.

12. Pharmaceutical compositions for the treatment of anaemias due to iron deficiencies, characterised in that they contain, as active principle, a compound according to claims 3 to 5.

13. Pharmaceutical compositions according to claim 12 in the form of single-dose sachets, syrups, phials or small bottles to be taken orally, tablets, capsules, sugar-coated pills and such like.

**Claims for the following Contracting State : ES**

1. A method of preparing proteins of vegetable or animal origin modified by the introduction into their structure of carboxylic groups able to combine with iron, characterised in that the introduction of carboxylic groups takes place through reaction of the proteins with an anhydride of an aliphatic dicarboxylic acid chosen from acetylaspartic acid and acetylglutamic acid.

2. A method according to claim 1, characterised in that the proteins are chosen from the proteins of powdered milk, precipitated or partially hydrolysed milk, egg, bovin serum, liver, fish, soya, extracts of bird and animal meat.

3. A method according to claims 1 and 2, characterised in that the proteins can be partially hydrolysed.

4. A method according to claims 1 to 3, characterised in that by strictly controlling the pH value, the proteins dissolved in water are reacted with an anhydride of an aliphatic dicarboxylic acid chosen from acetylaspartic acid and acetylglutamic acid.

5. A method according to claim 4, characterised in that the pH is maintained at a value of 7.5-8.5 by means of the addition of a base, where the base is an alkaline hydroxide or an alkaline carbonate or bicarbonate.

6. A method according to claims 4 and 5, characterised in that the base is a sodium hydroxide, sodium carbonate or sodium bicarbonate.

7. A method of preparing compounds containing bioavailable iron, characterised in that the modified proteins obtained according to the method of claims 1 to 6, after their isolation or without isolating them, are treated with an iron derivative.

8. A method according to claim 7, characterised in that the iron derivative is ferric chloride.

9. A method according to claims 7 and 8, characterised in that the iron content in the compounds containing bioavailable iron is comprised between 0.1% and 20% by weight.

10. A method according to claims 7 to 9, characterised in that the product which is formed after the treatment of the modified proteins with an iron derivative is separated by lyophilisation or by spray dryer.

11. A method of preparing pharmaceutical compositions for the treatment of anaemias due to iron deficiencies, characterised in that the iron-containing compounds obtained according to the method of claims 7 to 10 are mixed with adjuvants and/or usual additives.

**Patentansprüche**

**Patentansprüche für folgende Vertragsstaaten : BE,DE,FR,GB,NL,SE**

1. Proteine pflanzlichen oder tierischen Ursprungs, die durch Einführung von Carboxylgruppen, die imstande sind, sich mit Eisen zu kombinieren, in ihre Struktur modifiziert sind, dadurch gekennzeichnet, daß die Einführung von Carboxylgruppen durch Umsetzen von Proteinen mit einem Anhydrid einer aliphatischen Dicarbonsäure ausgewählt aus Acetylasparaginsäure und Acetylglutaminsäure erfolgt.

2. Proteine nach Anspruch 1, dadurch gekennzeichnet, daß sie ausgewählt sind aus den Proteinen von Milchpulver, gefällter oder teilweise hydrolysierter Milch, Ei, Rinderserum, Leber, Fisch, Soja, Fleischextrakten von Geflügel und Tieren.

3. Verbindungen, die bioverfügbares Eisen enthalten, erhalten durch Umsetzen der modifizierten Proteine der Ansprüche 1 und 2 mit einem Eisenderivat.

4. Verbindungen nach Anspruch 3, dadurch gekennzeichnet, daß das Eisenderivat Eisen(III)-chlorid ist.

5. Verbindungen nach Anspruch 3, dadurch gekennzeichnet, daß der Eisengehalt darin 0,1 bis 20 Gew.% beträgt.

6. Proteine nach Anspruch 2, dadurch gekennzeichnet, daß sie teilweise hydrolysiert sein können.

7. Verfahren zur Herstellung von Proteinen der Ansprüche 1 und 2, dadurch gekennzeichnet, daß die in Wasser gelösten Proteine unter strenger Kontrolle des pH-Wertes mit einem Anhydrid einer aliphatischen Dicarbonsäure ausgewählt aus Acetylasparaginsäure und Acetylglutaminsäure reagieren gelassen werden.

8. Verfahren nach Anspruch 7, dadurch gekennzeichnet, daß der pH durch Zusatz einer Base bei einem Wert von 7,5 bis 8,5 gehalten wird, wobei die Base ein Alkalimetallhydroxid oder ein Alkalimetallcarbonat oder -bicarbonat ist.

9. Verfahren nach den Ansprüchen 7 und 8, dadurch gekennzeichnet, daß die Base Natriumhydroxid, Natriumcarbonat oder Natriumbicarbonat ist.

10. Verfahren zur Herstellung von Proteinen der Ansprüche 3 bis 5, dadurch gekennzeichnet, daß die nach dem Verfahren von Anspruch 7 erhaltenen modifizierten Proteine nach ihrer Isolierung oder ohne Isolierung mit einem Eisenderivat behandelt werden und das gebildete Produkt durch Gefriertrocknen oder mit einem Sprühtrockner abgetrennt wird.

11. Verfahren nach Anspruch 10, dadurch gekennzeichnet, daß das Eisenderivat Eisen(III)-chlorid ist.

12. Pharmazeutische Zusammensetzungen zur Behandlung von durch Eisenmangel hervorgerufenen Anämien, dadurch gekennzeichnet, daß sie als Wirkstoff eine Verbindung nach den Ansprüchen 3 bis 5 enthalten.

13. Pharmazeutische Zusammensetzungen nach Anspruch 12 in Form von Säckchen mit einer einzigen Dosis, Sirupen, Ampullen oder kleinen Trinkampullen, Tabletten, Kapseln, Dragées und dgl.

**Patentansprüche für folgenden Vertragsstaat : ES**

1. Verfahren zur Herstellung von Proteinen pflanzlichen oder tierischen Ursprungs, die durch Einführung von Carboxylgruppen, die imstande sind, sich mit Eisen zu kombinieren, in ihre Struktur modifiziert sind, dadurch gekennzeichnet, daß die Einführung von Carboxylgruppen durch Umsetzen von Proteinen mit einem Anhydrid einer aliphatischen Dicarbonsäure ausgewählt aus Acetylasparaginsäure und Acetylglutaminsäure erfolgt.

2. Verfahren nach Anspruch 1, dadurch gekennzeichnet, daß die Proteine ausgewählt werden aus den Proteinen von Milchpulver, gefällter oder teilweise hydrolysierter Milch, Ei, Rinderserum, Leber, Fisch, Soja, Fleischextrakten von Geflügel und Tieren.

3. Verfahren nach den Ansprüchen 1 und 2, dadurch gekennzeichnet, daß die Proteine teilweise hydrolysiert werden können.

4. Verfahren nach den Ansprüchen 1 bis 3, dadurch gekennzeichnet, daß die in Wasser gelösten Proteine unter strenger Kontrolle des pH-Wertes mit einem Anhydrid einer aliphatischen Dicarbonsäure ausgewählt aus Acetylasparaginsäure und Acetylglutaminsäure reagieren gelassen werden.

5. Verfahren nach Anspruch 4, dadurch gekennzeichnet, daß der pH durch Zusatz einer Base bei einem Wert von 7,5 bis 8,5 gehalten wird, wobei die Base ein Alkalimetallhydroxid oder ein Alkalimetallcarbonat oder -bicarbonat ist.

6. Verfahren nach den Ansprüchen 4 und 5, dadurch gekennzeichnet, daß die Base Natriumhydroxid, Natriumcarbonat oder Natriumbicarbonat ist.

7. Verfahren zur Herstellung von bioverfügbares Eisen enthaltenden Verbindungen, dadurch gekennzeichnet, daß die nach dem Verfahren der Ansprüche 1 bis 6 erhaltenen modifizierten Proteine nach ihrer Isolierung

EP 0 243 322 B1

oder ohne Isolierung mit einem Eisenderivat behandelt werden.

8. Verfahren nach Anspruch 7, dadurch gekennzeichnet, daß das Eisenderivat Eisen(III)-chlorid ist.

9. Verfahren nach den Ansprüchen 7 und 8, dadurch gekennzeichnet, daß der Eisengehalt in den bioverfügbares Eisen enthaltenden Verbindungen 0,1 bis 20 Gew.% beträgt.

10. Verfahren nach den Ansprüchen 7 bis 9, dadurch gekennzeichnet, daß das Produkt, das nach Behandlung der mit einem Eisenderivat modifizierten Proteine gebildet wird, durch Gefriertrocknung oder mit einem Sprühtrockner abgetrennt wird.

11. Verfahren zur Herstellung von pharmazeutischen Zusammensetzungen zur Behandlung von durch Eisenmangel hervorgerufenen Anämien, dadurch gekennzeichnet, daß die nach dem Verfahren der Ansprüche 7 bis 10 erhaltenen eisenhaltigen Verbindungen mit Adjuvantien und/oder üblichen Additiven gemischt werden.